Europäisches Patentamt

(19) European Patent Office

Office européen des brevets

(11) Publication number: **0 168 651**
**A1**

## (12) EUROPEAN PATENT APPLICATION

(21) Application number: **85107458.3**

(22) Date of filing: **14.06.85**

(51) Int. Cl.⁴: **C 07 K 15/00**, C 12 P 21/02,
C 07 K 3/28, C 12 N 15/00,
A 61 K 37/02
// (C12P21/02, C12R1:91)

(30) Priority: **15.06.84 NL 8401911**

(43) Date of publication of application: **22.01.86**
**Bulletin 86/4**

(84) Designated Contracting States: **AT BE CH DE FR GB IT**
**LI LU NL SE**

(71) Applicant: **Stichting REGA V.Z.W.,**
**Minderbroedersstraat 10, B-3000 Leuven (BE)**

(72) Inventor: **Billiau, Alfons, Jachthuislaan 27, B-3230 Linden**
**(BE)**
Inventor: **De Somer, Pieter, Brusselsesteenweg 9,**
**B-3000 Leuven (BE)**
Inventor: **Van Damme, Jozef, Tintorettostraat 32,**
**B-1040 Brussel (BE)**
Inventor: **De Ley, Marc, Herendreef 12, B-3030 Heverlee**
**(BE)**

(74) Representative: **Bruin, Cornelis Willem et al,**
**Octrooibureau Arnold & Siedsma Isartorplatz 5,**
**D-8000 München 2 (DE)**

(54) Antiviral protein, method for producing the same and pharmaceutical compositions containing such protein.

(57) A protein, called IMP, recovered from the culture fluid of mitogen-stimulated leukocytes and purified until molecular homogeneity, appears to have a broad-spectrum antiviral activity similar to but slightly different from that of interferonbeta. It has a molecular weight of about 17000 daltons and a specified amino acid sequence. Methods of producing this protein and pharmaceutical utilisations thereof are suggested.

Antiviral protein, method for producing the same and
pharmaceutical compositions containing such protein.


This invention relates to a new
antiviral protein recovered from the culture fluid of
mitogen-stimulated leukocytes. Further, this invention
relates to methods for producing such protein and to
pharmaceutical compositions containing that protein.

It is known that leukocytes may be
stimulated by so-called mitogenic substances to grow
in a culturing medium and to show cell fission
therein. Further, it is known that the culture fluid
of leukocytes stimulated in this way will have a
certain antiviral activity, for if this culture fluid
is used for treating cells in a tissue culture, then
such cells will become resistant against subsequent
virous infections. It has been found that a first
carrier of the antiviral activity in said culture
fluid is interferon-gamma, a glycoprotein of about
45.000 daltons molecular weight. Further, another
protein-like material, called 22K, was found in
addition thereto. The latter protein could be
separated from interferon-gamma by means of gel
filtration and migrated then in the normal position
of molecules of 22.000 daltons. Uptill now, however,
the active component of 22K was not obtained with
sufficient purity to unambiguously determine its
molecular structure and the biological
properties connected therewith. Compare J. van Damme
et al., Eur. J. Immunol. 11, 937-942 (1981).

0168651

As a result of further research, it has now been found that this component may be isolated by means of chromatographic methods and that it is a protein having a molecular weight of about 17.000 daltons. This protein, called IMP, appears to be sufficiently pure and homogeneous for determination of its molecular structure. Thus, it has been established that the amino acid sequence (first 9 amino acids counted from the terminal amino acid carrying a free amino group) was: N.C.-proline-valine-glutamine-phenylalanine-threonine-asparagine-alanine-threonine (wherein N.C. = unclear). Further, the product IMP appears to have clear antiviral properties similar to but slightly different from those of interferon-beta. This fact means that a new source for the treatment of virous diseases has become available.

In the above-mentioned amino acid sequence, there is a possibility of variation in position 1 and a minor chance of variation in other positions. Further, it is possible that in future, variants and modifications of IMP may be found which have a similar, a stronger or a more selective antiviral activity. Therefore, variants and modifications of IMP are also included by the invention.

Thus, in a first aspect, the invention provides an antiviral protein called IMP and characterized by a molecular weight of about 17.000 daltons (determined by means of electrophoresis in a sodium dodecylsulphate-containing polyacrylamide gel), by an amino acid sequence (first 9 amino acids counted from the terminal amino acid carrying a free amino group) of: N.C.-proline-valine-glutamine-phenylalanine-threonine-asparagine-alanine-threonine (wherein N.C. = unclear), and by a broad spectrum antiviral activity similar to that of interferon-beta, as well as variants and modifications of such protein. Further, the invention provides methods for producing IMP and IMP-containing pharmaceutical compositions.

The invented protein IMP may be prepared in principle in different ways. A first preparation method preferred at the moment comprises culturing leukocytes in the presence of mitogenic substances and subjecting the resulting culture fluid to a concentration and purification treatment. However, the invention is not limited thereto since several other methods can be imagined.

The preferred method for preparing IMP (culturing leukocytes with stimulation by mitogenic substances followed by a concentration and purification treatment of the culture fluid) will now first be described in detail.

Any type of leukocytes or mixture of leukocytes may be used as a starting material provided that they can be stimulated by mitogenic substances. Leukocytes from peripheral blood which can be isolated easily from blood donations collected in blood transfusion centers are preferred. During isolation, a separation between leukocytes and erythrocytes should be accomplished and this can be effected by means of known methods, e.g. by treatment with an ammonium chloride solution followed by centrifuging; by dilution with an amylopectin derivative and a buffered saline solution, also followed by centrifuging; or else by centrifuging in a special plasmapheresis device; or the like. The resulting leukocytes will comprise a mixture of several cell types then and are capable of being stimulated by mitogenic substances. Further, the leukocytes might be isolated from human spleens or (if the analogous IMP from animals is desired) from the spleen or thymus of the corresponding animal species.

Several substances such as plant lectins including Concanavalin A (Con-A), Phytohemagglutinin (PHA-P), "pokeweed mitogen", lentil lectin and pea lectin; and bacteria extracts including Staphylococcal enterotoxin and staphylococcal culture supernatant and the like, can be used as mitogenic substances. Some of these mitogenic substances will have a stimulating activity only on certain types of leukocytes; in leukocyte mixtures, however, any mitogenic substance can be used.

The leukocytes and mitogenic substances may be combined in any suitable medium for cell culture or tissue culture. The medium RPMI 1640, developed in the Rosswell Park Memorial Institute, and comprising inorganic salts ($NaCl$, $NaHCO_3$, $NA_3HPO_4$, etc.), as well as glucose, several amino acids and several vitamins (compare J. Amer.Med. Ass., 199, 519-524 (1967)) is preferred. Several other substances may be added at will to this culture medium, e.g. fetal calf serum; chemical substances such as N-2-hydroxypiperazin-N-2-ethanesulfonic acid (HEPES) and N-Tris(hydroxymethyl)-methylglycine (Tricine); and the like (compare N.E. Good et al., Biochemistry, 5, 467-477 (1966) for the latter substances).

When the leukocytes have been combined with a mitogenic substance in a culture medium, they are incubated for some time at a suitable temperature ($37^{o}C$ for human leukocytes). The incubation period may be 0-5 days with an optimum around 48 hours. Thereafter, the culture fluid may be recovered and subjected to the required concentration and purification treatment. Several steps may normally be distinguished during purification and concentration of the culture fluid. Thus, a first step or series of steps may serve to remove the major part of the impurities and also to strongly diminish the fluid volume. The result thereof is a purified fluid having a high concentration of antiviral activity. A next step may serve to separate the antiviral substances as present (in this case interferon-gamma and IMP-containing material) from each other and a third step may be used for further fractionating the IMP-containing material and for recovering pure IMP.

Removal of the major part of the impurities under simultaneous reduction of the volume may well be effected with chromatographic methods. In principle, several methods are possible here. A purification in two steps, viz. first a chromatography on silicic acid followed by treatment with DEAE-Sephacel (a diethylaminoethyldextrane cross-linked with agarose) has given good results. In that case, the crude culture fluid is contacted with silicic acid at neutral pH for adsorbtion of desired substances from the fluid whereupon such substances may be eluted from the silicic acid, e.g. by means of a buffered solution of ethylene glycol, a buffered solution of tetraethylammonium acetate, a solution of glycine-HCl or the like. Next, the eluate may be contacted with DEAE-Sephacel at a pH of about 8.0 for adsorption of impurities to that material. After separation of the solid material, a highly purified and concentrated fluid may be recovered then. Further concentration is possible to intermediate or subsequent dialysis, normally against a buffered solution of polyethylene glycol.

In principle, the separation between interferon-gamma and IMP-containing material may be effected in any suitable way but it is preferred to use a gel filtration here. In that case, the purified fluid is added to a column of a molecular sieve whereupon the column may be eluted with an aqueous ethylene glycol solution. If the eluate is collected in fractions and if all fractions are tested on antiviral activity, two peaks in antiviral activity may easily be distinguished, such peaks corresponding to interferon-gamma and to IMP-containing material, respectively. Next, the fractions relating to these peaks may be pooled in groups.

-6-

Another possibility of separation is adsorption on a lectin column. Interferon-gamma will be adsorbed by such a column but IMP will not be adsorbed and the residual fluid after contact with the lectin column will then be an IMP-containing material.

In general, fractionating of the IMP-containing material may also be effected in any suitable way. The method of Fast Liquid Protein Chromatography wherein the fluid material is passed under a relatively high pressure through a cation exchanger column is preferred at the moment. This method may proceed rather rapidly thanks to the use of pressure, although in principle, an operation at normal pressure is also possible. Elution may be effected here with a concentration gradient of a saline solution. If the resulting fractions are tested on antiviral activity, the median fractions will show a clear activity peak resulting from the IMP. A purified IMP-solution may be obtained then by combining such fractions.

The four-step purification method here described (chromatography on silicic acid, adsorption to DEAE-Sephacel, fractionation by gel filtration and fractionation by FPLC) is illustrated in the following examples. It will be clear, that this purification method is only a preferred method and that many variants thereto can be imagined.

A modified purificationmethod can be effected if anti-IMP antibodies are available. In that case, fractionation by FPLC may be substituted by chromatography on a column which comprises such antibodies in immobilized form. The IMP will be adsorbed to the column then and may be eluted next with a suitable buffer solution. In general, the other steps may remain the same although the step of chromatography on silicic acid may perhaps be substituted by chromatography on porous glass beads (controlled pore glass). Perhaps, even a reduction in length of the purification treatment to result in a two-step method (e.g. chromatography on porous glass beads or silicic acid, followed by chromatography on immobilized anti-IMP antibodies) or to result into a one-step method (chromatography on immobilized monoclonal anti-IMP antibodies) may be possible.

The anti-IMP antibodies may be polyclonal or monoclonal. Polyclonal antibodies may be obtained by injecting IMP into a test animal, thereupon bleeding the animal and recovering an antibodies-containing serum from the blood by means of suitable purification methods. Monoclonal antibodies will need a more complicated method comprising e.g. injecting the product IMP to a test animal, recovering a suspension of spleen cells from that test animal, fusing the spleen cells with tumor cells, culturing a certain cell line of hybrid cells from the fusion mixture by cloning and subcloning under continuous testing on antibody activity and finally using the cells line for antibody production in tissue culture or in mice. Many variants are possible here, of course.

Although the method of culturing leukocytes in the presence of mitogenic substances followed by purification and concentration of the resulting culture fluid is preferred at the moment, the product IMP may also be prepared in other ways.

In such another way of preparation, a continuous cell line of hybrid cells derived from leukocytes and tumor cells is made first, whereupon the hybrid cells are cultured in a suitable culturing medium in the presence of mitogenic or other substances (e.g. phorbol esters) and the resulting culture fluid is subjected to a purification and concentration treatment. The continuous cell line may be made in a conventional way by subjecting the leukocytes (derived e.g. from peripheral blood or from spleens) to a cell fusion with suitable tumor cells in polyethylene glycol and recovering a continuous cell line of desired properties

-8-

from the fusion mixture by means of cloning and subcloning.

Purification of the culture fluid from the hybrid cells may be effected in a similar way as for the leukocyte culture fluid. This purification will be somewhat simpler because the starting material is more homogeneous.

Recombinant-DNA methods are used in still another preparation method. The gen for production of IMP is isolated from leukocytes and is introduced into a certain expression system by means of a directed vector. This expression system may comprise prokaryotic cells (e.g. cells of Escherichia coli), eukaryotic micro-organisms (e.g. yeasts or fungi), or eukaryotic cells from higher animals (e.g. mammalian cells, bird cells or human tumor cells) at option. The resulting cells may be cultured further in a suitable culturing medium and the culture fluid resulting thereby may be subjected to a purification and concentration treatment. In that case, the purification treatment may proceed along the same lines as above or along other lines.

Utilisation of recombinant-DNA methods will also include the possibility to obtain variants and modifications of IMP because certain modifications in the IMP molecule may be effected by initiating mutations in specific positions of the IMP-producing gen. Such modifications may lead e.g. to the same antiviral activity at shorter molecular chain, to an intensified activity or to a more selective activity.

The invented protein IMP has a clear antiviral activity which is expressed in several cell types, such as e.g. skin or muscle cells and further in certain sublines of a human osteosarcoma cell line (MG-63). The activity is similar to that of interferon because it is specific to a certain cell type but non-specific to a certain virus. This antiviral activity is resistant against treatment with an acid (pH: 2), sodium dodecyl sulphate (0.1%) ribonuclease and DNA-ase, but is abrogated by treatment with trypsin. Further, the antiviral activity is abrogated by previous treatments of the cell substrate with antiserums specifically directed against IMP, but not abrogated by previous incubation with antiserums against interferon-alpha or interferon-gamma. The activity may also be inhibited by antiserums against interferon-beta, especially when the antiserum is present together with IMP on the cell substrate. These properties lead in the direction of a certain similarity with interferon-beta although the IMP is not identical thereto.

On the basis of the preceeding, protein IMP may be used with advantage for the preparation of pharmaceutical compositions and for treating virus diseases.

Pharmaceutical compositions containing the protein IMP as an active ingredient may have the form of powders, suspensions, solutions, emulsions and also the form of ointments and pastes and may be used for parenteral (intradermal, intramuscular, intrathecal and the like) injections, for oral, rectal, intravaginal and intranasal administration or for topical administration (e.g. to an injured skin, to mucosae or eyes). These compositions may be prepared by combining the active ingredient with pharmaceutically acceptable excipients which are conventionally used for this purpose. Such excipients may comprise aqueous or non-aqueous solvents, stabilizers, suspending agents, dispersing agents, wetting agents and the like and will be familiar to an expert in the pharmaceutical field. Further, the composition may comprise any suitable additives such as polyethylene glycol and, if desired, colorants, flavourings and the like.

The pharmaceutical compositions will normally comprise at least 0.1 % (weight/volume) of the active ingredient. The actual concentration will depend from the virus disease and from the selected way of administration. In general, this concentration will be between 0.1 % and 100 %.

The following examples relate to
-the preparation of a culture fluid from mitogen-stimulated leukocytes (1),
-the purification and concentration of such a culture fluid (2 and 3), and
-the antiviral activity of the resulting IMP (4 and 5).

## EXAMPLE 1

Preparation of a culture fluid from mitogen-stimulated leukocytes.

A leukocyte composition (buffy coats) obtained as a spontaneous sediment from human donor blood was used as a starting material. This composition had a volume of 1520 ml and a leukocyte content of $110 \times 10^9$ (98% viable cells). It was diluted with 1520 ml phosphate-buffered saline solution (PBS) (compare R. Dulbecco et al., Journ. Exp. Med. 99, 167-182, 1954) and 1520 ml Plasmasteril (a solution of 60 g/l O-(2-hydroxyethyl)-amylopectin hydrolysate and 9 g/l NaCl, supplied by Fresenius AG, Bad Homburg, F.R.G.). The mixture was filled into cylinders of 10 cm diameter and 40 cm height and left stationary therein for 30 minutes at 37 °C until a neat separation into two layers was obtained. The lower layer comprised red blood cells and the leukocytes were present in the upper layer. The upper layer was recovered by suction and centrifuged (100 rpm, 10 minutes) whereupon the clear supernatant was removed by decanting.

The cell sediment was washed twice by suspending it into 1000 ml PBS and resedimented by centrifuging (900 rpm, 10 minutes)

The washed sediment was resuspended in 166 ml PBS and 83 ml Plasmasteril, resedimented by centrifuging (900 rpm, 10 minutes), resuspended in 100 ml culture medium RPMI-1640 (concentration $96 \times 10^9$ cells; 98 % viable cells), and thereafter divided over three culture vessels of the type "Spinner Vessel" each containing 4000 ml of RPMI-1640, supplemented with 2% fetal calf serum, 6mM N-2-hydroxypiperazin-N-2-ethanesulfonic acid (HEPES) and 13mM N-tris (hydroxymethyl)-methylglycine (Tricine). The pH was 7.4.

The cells were stimulated by addition to the medium of 10 ug/ml Concanavalin A and incubated under gentle stirring conditions for 44 hours at 37 °C.

After cultivation of the cells, the culture fluid was recovered and clarified by centrifuging (2000 rpm, 20 minutes). The antiviral potency of this culture fluid was $10^{3.4}$ AVU/ml in a test on MG-63-cells and $10^{3.0}$ AVU/ML in a test on HEp-2 cells (Am.Type Cult. Coll. no. CCL-23) (AVU=antiviral units). Instead of storing the fluid, it was immediately subjected to a purification and concentration treatment.

EXAMPLE 2

Purification and concentration of culture fluid (first part).

The starting material for purification was 9000 ml culture fluid from mitogen-stimulated leukocytes, obtained in the way of example 1. After addition of 90 g dry silicic acid thereto, the fluid was stirred for 2 hours at 4°C, thereupon centrifuged (3000 rpm, 10 minutes) and decanted.

The silicic acid sediment was shaken

-12-

with 2000 ml PBS, again centrifuged (3000 rpm, 5 minutes), decanted, shaken with 1000 ml of 1M NaCl in PBS, and again sedimented by centrifuging (3000 rpm, 5 minutes).

The washed silicic acid sediment was eluted 4 times with a tetraethylammoniumacetate buffer (TEAC). Each elution comprised the following steps: suspending in 125 ml of 0.4 M TEAC in PBS, supplemented with 0.5 mg/ml human plasma protein fraction (HPPF) (cohn-fraction V), stirring at 4°C for 30 minutes, followed by centrifuging (2.5 minutes, 3000 rpm) and recovering of the clear supernatant fluid. The resulting fluids were pooled to 500 ml of "TEAC eluate".

Next, the silicic acid sediment was eluted 4 times with a glycine buffer. Each elution comprised the following steps: suspending in 80 ml of 0.3 M glycine-HCl of pH = 2 supplemented with 0.5 mg/ml HPPF, stirring for 30 minutes at 4 °C, followed by centrifuging (2.5 minutes, 3000 rpm) and recovering the clear supernatant fluid. The resulting fluids were pooled to 320 ml of "glycine eluate".

The "TEAC eluate" and the "glycine eluate" were subjected separately to the following treatment:
-dialysis for 16 hours against 5000 ml of polyethylene glycol solution (15% PEG in 20mM Tris-HCl, pH = 8.0) resulting into 82 ml of "TEAC concentrate" and 40 ml of "glycine concentrate",
-dialysis for 6 hours against 2 x 5000 ml of Tris solution (20mM Tris-HCl, pH 8.0),
-stirring for 20 minutes at 4°C with 10 % (v/v) DEAE-Sephacel in 20 mM Tris-HCl (pH 8.0),
-centrifuging (4000 rpm, 20 minutes), and recovering of the supernatant fluid.

After sampling for testing the antiviral

-13-

activity, the resulting solution was stored at
-70°C.

The volume, the total amount of antiviral
activity and the total amount of protein was determined
for all fluids during this purification treatment.
Determination of the antiviral activity was effected
by measuring the inhibition of the cytopathic effect
(compare example 4) and determination of the amount
of protein was effected with the "BIO-RAD protein
Assay", as suggested by BIO-RAD laboratories,
Munich, F.R.G. The results are given in Table 1.

## Table 1

| Fraction | Volume | Total Antiviral activity (AVU) | Total amount of protein (mg) |
|---|---|---|---|
| Culture fluid | 9000 | 28,800,000 | 18,900 |
| Fluid after Silicic acid treatment | 9000 | 7,200,000 | 14,400 |
| "TEAC eluate" | 500 | 12,500,000 | 300 |
| "Glycine eluate" | 320 | 3,200,000 | 224 |
| "TEAC concentrate" | 82 | 10,250,000 | 328 |
| "Glycine concentrate" | 40 | 2,560,000 | 204 |
| "TEAC concentrate" after Sephacel treatment | 82 | 8,200,000 | 16 |
| "Glycine concentrate" after Sephacel treatment | 40 | 2,000,000 | 4 |

## EXAMPLE 3

Concentration and purification (second part)

The starting material was a partially purified culture fluid obtained in the way of example 2 with the exception that the silicic acid had been eluted with a solution of 50% (v/v) ethylene glycol in 1.4 M NaCl/PBS at pH 7.4 and that the fluid was contained in 45 ml Tris-HCl of pH 8.0. The fluid was concentrated to 5 ml by dialysis against 15% polyethylene glycol in PBS.

The concentrated fluid was loaded on a column (2.6 cm diameter x 100 cm height) of a molecular sieve (Ultrogel AcA-54 from Réactifs IBF, Villeneuve-la-Garenne, France), whereupon the column was eluted in 5 ml portions with a solution of 1% (v/v) ethylene glycol in 1.55 M NaCl of pHH 7.2. All resulting fractions were analyzed on protein content and antiviral activity. Fractions nrs. 66 to 76, which contained an antiviral activity peak at a molecular weight around 22.000 daltons, were pooled to 50 ml, concentrated by dialysis against 500 ml of 15% polyethylene glycol in 50mM sodium formiate of pH 4.0, and further concentrated by dialysis against 2 x 500 ml of the same buffer solution without polyethylene glycol. This resulted into 8 ml of purified material.

The purified material was subjected to Fast Protein Liquid Chromatography by loading it under pressure to a column of cation-exchanger (Mono-S from Pharmacia, Uppsala, Sweden). Elution was effected with 50 portions of 1 ml eluting agent which contained a gradient of NaCl (0 to 0.65 M) in 50 mM sodium formiate of pH 4.0. The fractions 35 to 38 were pooled to 54 ml of product (IMP).

The volume, the total antiviral activity and total amount of protein was determined for all fluids during this purification treatment (the starting solution, the material after gel filtration and the product after FPLC). Determination of the antiviral activity was effected in the way of example 4 and determination of the amount of protein was effected by "BIO RAD protein assay". The results are given in table 2 wherein "N.D." has the meaning of: non-detectable (the amount of IMP was less than 0.01 mg/ml). With regard to the antiviral activity of the starting solution, it should be noted that this solution did not only contain IMP but interferon-gamma as well.

For determination of molecular weight, the total amount of 4 ml IMP was further concentrated and thereafter subjected to electrophoresis on sodium dodecyl sulphate-containing polyacrylamide gel (compare Nature 227, 680-685 (1970)). It appeared that the antiviral activity and the capability to be stained by Coomassie-Blue were concentrated in a single band of molecular weight 17,000 daltons.

The amino acid sequence of the first 9 amino acids, counted from the terminal amino acid carrying a free amino group, was determined by stepwise degradation according to Edman in a gas phase sequenator and was as follows: (N.C.-proline-valine-glutamine-phenylalanine-threonine-asparagine-alanine-threonine) (wherein N.C. = unclear). It should be noted here that a detection of cysteine is impossible with said determination method, meaning that there is a rather small chance of cysteine being co-present.

## Table 2

|  | Volume | Total Antiviral Activity (AVU) | Total amount of protein (mg) |
|---|---|---|---|
| Starting solution | 5 | 1,600,000 | 5.5 |
| Material after gel filtration | 50 | 1,000,000 | 0.1 |
| Product (IMP) after FPLC | 4 | 160,000 | N.D. |

EXAMPLE 4

Determination of antiviral activity

In this example, the antiviral activity of the product was measured by inhibition of the cytopathic effect (CPE), that is by utilising the capability of the product to prevent destruction of sensitive cells due to a virus infection.

An IMP composition, purified in the way of the preceeding examples, was diluted stepwise (in steps of 0.5 $\log_{10}$). The diluted compositions were added to human diploid fibroblast cells in the wells (0.6 cm diameter) of a microtiter plate. After 16 hours of incubation at 37 °C, a test virus was added thereto. The occurrence of a cytopathic effect was monitored by microscopic examination. As soon as this effect was apparent in cells that had not been treated with the IMP composition, all cells were stained for 2 hours with a suitable dyestuff (neutral red, 0.17 g/100 ml, diluted in 1/30 in PBS). After removal of excess dyestuff, the cells were extracted with a mixture of acid and alcohol (sodium citrate buffer of pH 4.2, and ethanol in a ratio of 50 : 50 (v/v)), whereupon the optical density of the extracts was measured at 540 nm wavelength. The results were plotted against the dilution and the "end point dilution" was determined by projecting the 50 % dyestuff uptake points of the curves on the axis where the dilution was plotted on a logaritmic scale. The antiviral potency was expressed as antiviral units/ml (AVU/ml) wherein $\log_{10}$ AVU/ml = $-\log_{10}$ "end point dilution". The results are given in table 3 which includes values for positive control samples of interferon-alpha, interferon-beta and interferon-gamma.

Viruses from three different families were used as test virus, viz.: vesicular stomatitis virus (VSV, Rhabdoviridae family), Semliki Forest virus (SFV, Togaviridae family), and mengovirus and encephalomyocarditis virus (mengo and EMC, Picornaviridae family).

It can be seen from the table that the IMP composition as well as the various interferon types were active against all viruses. Moreover, it appears that SFV, which was the least sensitive virus to interferon, was also the least sensitive to IMP:

## EXAMPLE 5

### Determination of antiviral activity

In this example, the antiviral activity of an IMP composition was determined by its abiliy to inhibit the synthesis of viral RNA. This was done by specifically blocking the cellular RNA synthesis by addition of actinomycin D and measuring $^3$H-uridine in corporation in the acid-precipitable material.

An IMP composition from the preceeding examples was diluted in steps of 0.5 $\log_{10}$. The resulting dilutions were added to human diploid fibroblast cells which were present in the wells (0.6 cm diameter) of a microtiter plate. After incubation for 16 hours at 37°C, a test virus was added (an average of 10 viable virus particles per cell) in the presence of 1 ug/ml actinomycin D. After incubation for 3 hours, 5 ugCi/ml $^3$H-uridine was added and after another 3 hours, the incorporation of $^3$H-uridine was measured. The dilution which reduced the $^3$H-uridine incorporation to 50 % of the incorporation in non-IMP-treated cells was taken as an "end point dilution". The antiviral potency of the samples was expressed in AVU/ml, wherein $\log_{10}$ AVU/ml = $-\log_{10}$ "end point dilution". The results for 4 different test viruses are given in table 3, thereby using interferon-alpha, interferon-beta and interferon-gamma again for comparison.

It can bee seen from the table that the antiviral effect of IMP was present with all four test viruses belonging to three different taxonomic families.

Table 3

| Effect measured | Virus | Antiviral activity ($\log_{10}$AVU/ml) | | | |
|---|---|---|---|---|---|
| | | HuIFN- | HuIFN- | HuIFN- | IMP |
| CPE inhibition | VSV | 4.0 | 4.8 | 3.0 | 3.2 |
| (Example 4) | EMC | 3.8 | 4.7 | 3.0 | 3.5 |
| | Mengo | 3.7 | 4.4 | 2.8 | 1.8 |
| | SFV | 2.8 | 3.5 | 2.0 | 1.5 |
| Reduction of | VSV | 3.3 | 4.0 | 2.9 | 3.9 |
| viral RNA | EMC | 3.5 | 4.2 | 2.9 | 4.4 |
| synthesis | Mengo | 3.4 | 4.2 | 3.0 | 4.3 |
| (Example 5) | SFV | 3.6 | 4.1 | 3.2 | 4.1 |

0168651

CLAIMS

1. Antiviral protein called IMP and characterized by a molecular weight of about 17,000 daltons (determined by electrophoresis in a sodium dodecyl sulphate-containing polyacrylamide gel), by an amino acid sequence (first 9 amino acids counted from the terminal amino acid carrying a free amino group) of: N.C.-proline-valine-glutamine-phenylalanine-threonine-asparagine-alanine-threonine (wherein N.C. = unclear), and by a broad-spectrum antiviral activity similar to that of interferon-beta, as well as variants and modifications of that protein.

2. The antiviral protein of claim 1, characterized in that its antiviral activity is resistant against treatment with acid, sodium dodecyl sulphate, ribonuclease and DNA-ase and that it is abrogated by treatment with trypsin.

3. A method of preparing IMP, characterized by culturing leukocytes in the presence of mitogenic substances and subjecting the resulting culture fluid to a concentration and purification treatment.

4. The method of claiim 3, characterized in that said leukocytes have been derived from peripheral blood.

5. The method of claim 3, characterized in that said mitogenic substances are plant lectins or bacterial extracts.

6. The method of claims 3-5, characterized by effecting the concentration and purification of the culture fluid in a number of steps.

7. The method of claim 6, characterized in that the concentration and purification treatment comprises one or more steps for removing most of the impurities, a step for separating the antiviral substances as far as present, and a step for recovering pure IMP.

8. The method of claim 7, characterized in that the step for separating the antiviral substances is effected by gel filtration.

9. The method of claim 7, characterized in that the step for recovering pure IMP is effected by "Fast Protein Liquid Chromatography".

10. The method of claim 7, characterized in that the step of recovering pure IMP is effected by chromatography on immobilized anti-IMP-antibodies.

11. The method of claim 6, characterized in that said purification treatment comprises a step for removing most of the impurities and a step for recovering pure IMP by chromatography on immobilized anti-IMP-antibodies.

12. The method of claim 3, characterized in that said concentration and purification treatment is effected by chromatography on immobilized monoclonal anti-IMP-antibodies.

13. A method of preparing IMP, characterized by culturing hybrid cells derived from leukocytes and tumor cells in the presence of mitogens or other substances and subjecting the resulting culture fluid to a concentration and purification treatment.

14. A method of preparing IMP, characterized by culturing cells of an expression system having incorporated therein the IMP-producing gen of leukocytes in a suitable culturing medium, in the presence of or without mitogenic or other substances, and subjecting the resulting culture fluid to a concentration and purification treatment.

15. The method of claim 14, characterized by preparing a variant or modification of IMP by modification of said gen.

16. A pharmaceutical composition having antiviral activity and characterized by an effective amount of IMP together with or without a pharmaceutically acceptable excipient.

European Patent
Office

**EUROPEAN SEARCH REPORT**

0168651
Application number

EP 85 10 7458

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| D,A | CHEMICAL ABSTRACTS, vol. 96, no. 13, 29th March 1982, page 536, no. 102221x, Columbus, Ohio, US; J. VAN DAMME et al.: "Inferferon induced in human leukocytes by concanavalin A: isolation and characterization of gamma- and beta-type components" & EUR. J. IMMUNOL. 1981, 11(11), 937-42 * Abstract * | 1-7,16 | C 07 K 15/00<br>C 12 P 21/02<br>C 07 K 3/28<br>C 12 N 15/00<br>A 61 K 37/02 //<br>(C 12 P 21/02<br>C 12 R 1:91 ) |
| A | CHEMICAL ABSTRACTS, vol. 99, no. 11, 12th September 1983, page 431, no. 86417v, Columbus, Ohio, US; J. VAN DAMME et al.: "An interferon-beta-like or interferon-inducing protein released by mitogen-stimulated human leukocytes" & J. GEN. VIROL. 1983, 64(8), 1819-22 * Abstract * | 1-7,16 | |
| A | WO-A-8 300 693 (A/S ALFRED BENZON) * Claims 1,6,22,24,37,38,50,59,72,73,83-85 * | 1-16 | **TECHNICAL FIELDS SEARCHED (Int. Cl.4)**<br><br>C 12 P<br>C 12 N<br>A 61 K |
| A | EP-A-0 108 585 (GENENTECH, INC.) * Claims 1,9,11; page 4, line 33 - page 5, line 4; example 1 * | 1-16 | |

--- -/-

The present search report has been drawn up for all claims

| Place of search | Date of completion of the search | Examiner |
|---|---|---|
| THE HAGUE | 23-09-1985 | RYCKEBOSCH A.O.A. |

| DOCUMENTS CONSIDERED TO BE RELEVANT | | | Page 2 | |

| Category | Citation of document with indication, where appropriate, of relevant passages | Relevant to claim | CLASSIFICATION OF THE APPLICATION (Int. Cl.4) |
|---|---|---|---|
| A | DE-A-3 325 131 (DAMON BIOTECH, INC.)<br>* Claims 1,2,5,6,14 *<br><br>----- | 1,2,14,16 | |

TECHNICAL FIELDS SEARCHED (Int. Cl.4)

The present search report has been drawn up for all claims

| Place of search | Date of completion of the search | Examiner |
|---|---|---|
| THE HAGUE | 23-09-1985 | RYCKEBOSCH A.O.A. |